# EUROPEAN PATENT APPLICATION

(11) **EP 4 159 201 A1**
(43) Date of publication of application: **05.04.2023**
(21) Application number: 21218287.7
(22) Date of filing: 30.12.2021
(51) Int. Cl.: A61K 9/19, A61K 9/50, A61P 25/26

(54) **ENCAPSULATED MICROPARTICLES AND NANOPARTICLES OF DIMETHYLTRIPTAMINES**

(30) Priority: 30.09.2021 UY 39446; 27.12.2021 US 202163293975 P
(71) Applicant: BIOMIND LABS INC, Toronto, Ontario M5J 2T9 (CA)
(72) Inventor: ANTALICH, Alejandro, Pando, Canelones (UY)
(74) Representative: Padial Martinez, Ana Belen

(57) **Abstract**

The present invention relates to a process of obtaining encapsulated microparticles and/or nanoparticles of DMT/5-MeO-DMT, or pharmaceutically acceptable salts or derivatives thereof. Pharmaceutical compositions comprising the particles can further include an inhibitor of the Monoamine oxidase (MAOI), and are useful for the personalized treatment of neurological and/or psychiatric disorders, and/or inflammatory disorders.

## Description

### FIELD OF THE INVENTION

The present invention refers to a process of obtaining encapsulated microparticles and/or nanoparticles of N, N-dimethyltryptamine (DMT) or 5-methoxy-N, N-dimethyltryptamine (5-MeO-DMT), or pharmaceutically acceptable salts or derivatives thereof. The encapsulated particles can be used in various pharmaceutical formulations for the personalized treatment of neurological, psychiatric and/or inflammatory disorders.

### SUMMARY

One general aspect of the invention discloses a composition that includes:
particles of:
   i) N,N-dimethyltryptamine (DMT), 5-methoxy-N,N-dimethyltryptamine (5-MeO-DMT), or pharmaceutically acceptable salts or derivatives thereof; and
   ii) a pH-sensitive polymer,
wherein the particles are nanoparticles or microparticles.

In this embodiment, the composition can include a therapeutically effective dose of the DMT or 5-MeO-DMT.

In an embodiment of the invention, the composition can include an inhibitor of monoamine oxidase (MAOI), and the MAOI can be selected from beta-carbolines, resveratrol, curcumin, flavonoids, quercetin, crysine, shawls, and pharmaceutically acceptable salts or derivatives thereof.

In an embodiment of the invention, the weight ratio of i) to ii) can be between 1:100 to 1:4, and in an exemplary embodiment the weight ratio of i) to ii) can be between 1:10 to 1:4.

In an embodiment of the invention, the pH-sensitive polymer is soluble at a pH of greater than 6.

In an embodiment of the invention, the particles can have a particle size distribution of between 70 nm to 200 µm, and in an exemplary embodiment the particles can have a particle size distribution of between 70 nm and 300 nm.

In an embodiment of the invention, the composition can further include a surfactant, and in an exemplary embodiment the surfactant can be Tween 80.

Another embodiment of the invention discloses a method of treating a neurological and/or psychiatric disorder and/or inflammatory disorder in a patient in need thereof by administering to the patient the composition.

Another embodiment of the invention discloses a method of preparing an encapsulated dimethyltryptamine, the method including the steps of:
i) dissolving a pH-sensitive polymer in one or more solvents to make a first solution;
ii) agitating the first solution while adding DMT, 5-MeO-DMT, or a pharmaceutically acceptable salt or derivative thereof to make a second solution;
iii) prepare a separate aqueous third solution, which can optionally include a dissolved surfactant;
iv) agitating the third solution while adding the second solution to the third solution to obtain a fourth solution;
v) evaporating the one or more solvents from the fourth solution to obtain an aqueous intermediate;
vi) freezing the aqueous intermediate at -60 °C to obtain a frozen solid; and
vii) lyophilizing the frozen solid to obtain the encapsulated dimethyltryptamine.

Another embodiment of the invention discloses a pharmaceutical dosage form that includes the composition. The pharmaceutical dosage form can be a suspension, a granulated capsule, a soft gel capsule, a hard gel capsule, or a tablet.

### BACKGROUND OF THE INVENTION

Both N, N-dimethyltryptamine (DMT) and 5-methoxy-N, N-dimethyltryptamine (5-MeO-DMT) belong to the psychoactive indole alkylamine family and are naturally biosynthesized by various natural organisms.¹

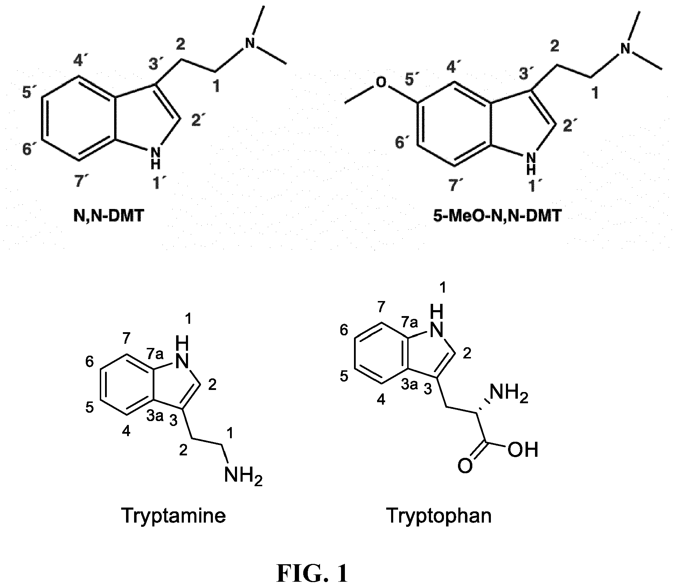

As can be seen from their atomic structures **(****Figure 1****),** both DMT and 5-MeO-DMT are molecular analogs of tryptamine (3-(2-Aminoethyl) indole, 2-(3-Indole) ethylamine), which itself is a molecular analog of tryptophan. Tryptophan is an essential amino acid that comes from the diet in animals and is produced endogenously in plants. Several cultures used plants containing DMT and 5-MeO-DMT for medicinal, psychological and entheogenic purposes for at least 4 millennia.^{2,3}

More recently, both DMT and 5-MeO-DMT have become the subject of an increasing number of scientific studies and clinical trials for the treatment of a variety of psychiatric disorders, such as depression, anxiety, headache, and obsessive-compulsive disorder.^{4,5,6,7,8} These therapeutic effects are the result of both drugs acting as non-selective serotonin agonists at the 5-HT2A, 5-HT2C, 5-HT1A receptors, among others. The 5-HT system is commonly associated with cognition, memory, emotions, circadian rhythm, alertness, and pain inhibition.^{9,10,11,12} Scientific studies have shown that in humans both DMT and 5-MeO-DMT are endogenously synthesized by the retina and pineal gland, and that they have also been detected in blood, urine, and cerebrospinal fluid.^{13,14,15,16,17}.

Both compounds are thermolabile, photodegradable and easily oxidizable molecules, and therefore, it is necessary to develop pharmaceutical formulation strategies that prevent degradation or decomposition. For example, metabolically, both molecules suffer from the first-pass effect due to deamination mediated by the enzyme monoamine oxidase A (MAO-A), resulting in their inactivated when orally administered. In order to make oral administration a viable delivery route for dimethyltryptamine, dosage forms thereof must be devised which avoid or minimize this first-pass effect.^{18,19,20}

One must also consider pharmacokinetics in developing delivery systems; specifically, taking into account their rapid onset of action and relatively short duration. An individual's rate of metabolization of these tryptamines impacts the pharmacodynamics; that is, the physiological, biochemical, and molecular effects of the DMT and/or 5-MeO-DMT. In case of psychedelic substances, this impacts the quality of the acute psychedelic experience and, therefore, influences the therapeutic result. People metabolize drugs at different speeds, which is due to many factors, such as the speed at which the drug is transported to cells, the rate at which the drug decomposes within cells, and the speed at which the drug is excreted. Genetic characteristics also influence these processes since they are mediated by enzymes and transport proteins. Environmental factors also play an important role. Diet, for example, can increase the production of a relevant enzyme or provide cofactors that modulate the activity of different enzymes, particularly when administered orally.²¹

There is a continuing need to design and develop pharmaceutical formulations that employ strategies to protect these molecules from degradation upon administration to improve bioavailability and prolong circulation time in the body, thereby increasing duration of action as well as therapeutic efficacy.

The present invention is directed toward strategies leading pharmaceutical formulations of DMT and 5-MeO-DMT that preserve their bioactivity while also being suitable for oral administration. This is important, since, despite the difficulties associated with oral administration of these molecules, oral administration has a number of advantages, such as its comfort and safety, which make it the preferred mode of drug administration by patients.²²

### BRIEF DESCRIPTION OF THE DRAWINGS

Figure 1 shows the chemical structures of DMT and its analogue 5-methoxy-N, N-dimethyltryptamine (5-MeO-DMT), as well as tryptamine and tryptophan.

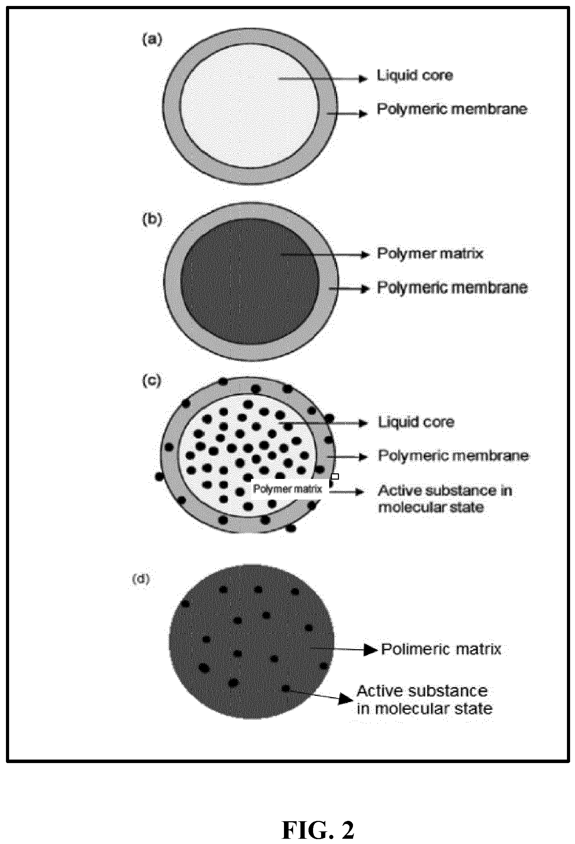

Figure 2 shows a graphic scheme of microparticles and nanoparticles. (a) shows a spherical nanoparticle comprising a solid polymeric membrane surrounding a liquid phase core. In this case the active compound is dissolved in the core. (b) shows a spherical nanoparticle comprising a solid polymeric membrane surrounding a solid phase core. In this case, the active compound is homogeneously dispersed in the core. (c) shows a spherical nanoparticle comprising a solid polymeric membrane surrounding a liquid phase core. In this case, the active substance is distributed both in the liquid phase of the core and in the wall material. In this case, the drug can be adsorbed from the surface. (d) shows a spherical nanoparticle formed by a solid polymeric material. In this case, the active compound is homogeneously dispersed throughout the nanoparticle.

### DETAILED DESCRIPTION OF THE INVENTION

The term "and/or" as used herein is defined as the possibility of having one or the other or both. For example, "A and/or B" provides for the scenarios of having just A or just B or a combination of A and B. If the claim reads A and/or B and/or C, the composition may include A alone, B alone, C alone, A and B but not C, B and C but not A, A and C but not B or all three A, B and C.

As used herein, the term "pharmaceutically acceptable salts or derivatives thereof" herein refers to those salts or derivatives which possess the biological effectiveness and properties of the salified or derivatized compound and which do not produce adverse reactions when administered to a mammal, preferably a human. The pharmaceutically acceptable salts may be inorganic or organic salts; examples of pharmaceutically acceptable salts include but are not limited to carbonate, hydrochloride, hydrobromide, sulphate, hydrogen sulphate, citrate, maleate, fumarate, tifluoroacetate, 2-naphthalenesulphonate, and para-toluenesulphonate. Further information on pharmaceutically acceptable salts can be found in Handbook of pharmaceutical salts, P. Stahl, C. Wermuth, WILEY-VCH, 127-133, 2008. Pharmaceutically acceptable derivatives include esters, the ethers, and N-oxides.

Disclosed herein is an encapsulation system for pharmaceutical forms of DMT or 5-MeO-DMT, or pharmaceutically acceptable salts or derivatives thereof, that make oral administration feasible.

Encapsulation is the process by which active ingredients are trapped in an encapsulating material - also referred to as a wall material and is based on the ability of the wall material to trap or coat hydrophobic or hydrophilic molecules. Examples of particles generated by encapsulation techniques are shown in **(****Figure 2****).²³**

Encapsulation systems for the controlled release of drugs include biomaterials that can be both micrometric and nanometric. "Micrometric" refers to particles with sizes in the micrometer range, for example 0.5 µm to 200 µm, 0.5 µm to 100 µm, 1 µm to 100 µm, or 1 µm to 10 µm. "Nanometric" refers to particles with sizes in the nanometer range, for example 1 nm to 1000 nm, 1 nm to 500 nm, 1 nm to 100 nm, or 1 nm to 10 nm. The systems are used as drug delivery systems for the transport and controlled time release of the active ingredients they contain. These systems must be carefully designed based on the functionality they must have and the drug they will contain. For example, the functionality can include the release profile, such as the pH range over which the drug is or is not released, the solubility (hydrophobicity or hydrophilicity) of the active ingredient being encapsulated, and the nature of the drug being administered.

In the pharmaceutical industry, applications of this strategy have been increasing due to the need to encapsulate biologically active ingredients to protect them from factors such as heat, humidity and solar radiation, and degradation by external agents after administration (for example oxidation by reactive oxygen species). Encapsulation systems prolong the stability and increases the efficacy the bioactive molecules they contain by improving their bioavailability, biodistribution, and decreasing possible adverse effects.^{24,25}

Further, if the size of the drug particles is micrometric, the term microencapsulation is used, and if the size is nanometric, nanoencapsulation is used. Both micrometric and nanometric particles are contemplated herein and are generally referred to as microparticles or nanoparticles (also referred to herein collectively as micro/nanoparticles). Oral administration of both DMT and 5-MeO-DMT inactivates both compounds due to first-pass metabolism mediated by the enzyme monoamine oxidase. Typically, this problem is solved by administration of MAO inhibitors (MAOIs) in conjunction with the dimethyltryptamine compound.

In embodiments of the invention, encapsulation of DMT/5-MeO DMT or pharmaceutically acceptable salts or derivatives thereof, is done together with an MAOI. Non-limiting examples of MAOIs include beta-carbolines, resveratrol, curcumin, or flavonoids, such as quercetin and crysine, or derivatives such as substituted shawls or analogues of curcumin and resveratrol, among others.

In general, the MAOIs act by inhibiting the activity of the enzyme monoamine oxidase, thereby preventing degradation of monoamine neurotransmitters. In the case of DMT and 5-MeO-DMT the MAOI prevents deamination of the dimethyltryptamine.

MAOIs are naturally present in ayahuasca infusions, and their presence promotes the psychotropic effects of the infusions. According to the present invention, MAOIs of natural origin are preferred to avoid problems associated with the consumption of synthetic MAOIs.^{26,27,28}

Microparticle and nanoparticle encapsulation systems are well known in the art. EP0130162A2 teaches the simultaneous formation and encapsulation of small particles from aqueous solutions of compounds whose solubility is greater at a first pH than at a second pH. The process is preferably used to prepare a readily soluble encapsulated pharmaceutically active compound. GB2135954A describes microcapsules and their production process. EP0212751A2 teaches a process for the microencapsulation of a drug and pharmaceutical compositions thereof. DE19930795A1 teaches the encapsulation of active agents by diffusion of previously prepared particles in alginate to provide slow-release, injectable formulations. CN103054810A teaches a pharmaceutical composition of curcumin and albumin nanoparticles, a method of preparing them, and their use as antineoplastic drugs.

However, there are no microparticle and nanoparticle encapsulated formulations of DMT and/or 5-MEO-DMT, or pharmaceutically acceptable salts or derivatives thereof, with pH-sensitive polymers for the preparation of pharmaceutical forms. For example,WO2021003467A1 teaches compositions that include a compound derived from plants or fungi, or their synthetic equivalents. WO2021168082A1 teaches specific tryptamines for use in the treatment of mood disorders. WO2021016423A1 discloses compositions containing two purified toad secretion tryptamines chosen from 5-MeO-DMT and 5-MeO-NMT, among others. WO2021116503A2 teaches deuterated N,N-dimethyltryptamine and compositions comprising one or more deuterated N,N-dimethyltryptamines for use in therapy.

The methods disclosed herein achieve greater chemical stability of DMT and/or 5-MeO-DMT and/or pharmaceutically acceptable salts or derivatives thereof, thereby solving the instability problems of the molecules in order to increase their therapeutic efficacy and allow for co-encapsulation of more than one drug, thereby enabling co-administration of DMT/5-MeO-DMT, and/or its pharmaceutically acceptable salts or derivatives thereof, with an MAOI of natural origin to prevent inactivation by first-pass metabolism mediated by the enzyme monoamine oxidase (MAO).

Disclosed herein is a nano/micro encapsulation system of DMT/5-MeO-DMT or pharmaceutically acceptable salts or derivatives thereof to protect the molecules from degradation by external agents and avoid deactivation from first-pass metabolism. The invention also includes formulations comprising nano/micro encapsulated DMT/5-MeO-DMT or pharmaceutically acceptable salts or derivatives thereof.

The digestive tract contains a wide range of pH values. The most acidic, between 1 and 3, occurs in the stomach, and when descending into the digestive system, the pH increases to 5 to 7 in the small intestine, and reaches about 8 in the large intestine.

Enteric coatings, also known as pH-sensitive polymers, are gastro-resistant coatings that are applied to oral medications to control the location in the gastrointestinal tract where medication is absorbed. Typically, these polymers contains synthetic polyacid materials which dissolve based on the pH of the medium in which they are located, and therefore allow for the design of encapsulation systems that release the active ingredient contained therein when the wall material (i.e. the pH-sensitive polymer) dissolves in the desired area of the digestive tract. One type of pH-sensitive polymer is the Eudragit^{™} family of functional polymers.

Eudragit^{™} L-100 and S100 are copolymers of methacrylic acid and methyl methacrylate, and dissolve at a pH of greater than 6 by deprotonation of their carboxylic groups. This property allows them to be used for the development of systems that release active agents at the intestinal level, thereby avoiding potential degradation in the stomach.

Eudragit^{™} L100 dissolves at a pH in the range of 6 to 7 which causes the release of the drug to occur in the jejunum, while Eudragit^{™} S100 dissolves at a pH of greater than 7 for release in the ileum and colon. Alternative pH-sensitive polymers suitable for use in the present invention include, but are not limited to, cellulose acetate phthalate, cellulose acetate trimellitate, hydroxy propyl methyl cellulose phthalate, hydroxy propyl methyl cellulose acetate succinate, and polyvinyl acetate phthalate, among others. Depending on the pH at which the polymer dissolves, one can target where the active agent is released in the gastrointestinal tract.

The encapsulation system of the present invention also prevents and/or minimizes inactivation from first-pass metabolism mediated by the enzyme monoamine oxidase (MAO). Although the present invention can reduce problems associated with first pass metabolism, if necessary achieve this, the encapsulation system can optionally include an MAOI, such as quercetin, curcumin, or resveratrol, among others. The MAOI can be included in the encapsulation system, or pharmaceutical dosage forms comprising the encapsulation system. Alternatively, the MAOI can be administered in a separate pharmaceutical composition that is administered in combination with the dosage form containing the encapsulation system.

Micro/nanoparticles (or encapsulates, can be formed by at least an evaporationmethod or a precitpitation method, both of which are sczlable.. The first method for preparing the inventive encapsulates, a co-evaporation method, includes dissolving the active ingredient and the polymer wall material in the same solvent or mixture of solvents. This solution or mixture is then added to an aqueous solution to form the micro/nanoparticles droplets. The size of the micro/nanoparticles dispersed in the aqueous solution can be controlled by controlling the size of the droplets sprayed into the aqueous phase or by controlling the rate of agitation of the aqueous phase as the polymer/active agent solution or mixture is added. The aqueous solution can optionally include a surfactant to modify the surface tension between the aqueous medium and the micro/nanoparticles to prevent aggregation and further control the size of the micro/nanoparticles. Surfactants include, but are not limited to, polysorbates, such as Tween 80, Span (i.e. Sorbian), and poloxamers. The organic phase is then evaporated, for example under reduced pressure, and the water removed via lyophilization to obtain the solid microparticles or nanoparticles (see Figure 2(d)).

In an alternative micro/nano-precipitation method, an organic phase containing the active ingredient and the wall material is dripped or sprayed into a solution in which the drop solidifies by a physicochemical mechanism. In this case, particle size can be altered by controlling the size of the droplets sprayed/dripped onto the aqueous phase and / or adjusting the rate of agitation of the aqueous phase as the polymer / active ingredient solution or mixture is added.

After obtaining the solid by any of these methods, subsequent grinding and sieving steps can be added to refine and obtain the desired particle size distribution.

In exemplary embodiments, the microparticles or nanoparticles obtained have a weight ratio of active compound to wall material of between 1:100 and 1:10, and more preferably a ratio between 1:10 and 1:4. The particles obtained have a particle size distribution of between 70 nm to 200 µm, more preferably between 70 nm to 300 nm.

A general procedure for the preparation of an encapsulated dimethyltryptamine (e.g., DMT or 5-OMe-DMT) includes the following steps:
i) dissolve a pH-sensitive polymer in one or more suitable solvents to make a first solution;
ii) agitate the first solution and add the dimethyltryptamine to make a second solution;
iii) prepare a separate aqueous third solution, which can optionally include a dissolved surfactant;
iv) agitate the third solution while adding the second solution to the third solution to obtain a fourth solution;
v) evaporate the one or more suitable solvents from the fourth solution to obtain an aqueous intermediate;
vi) freeze the aqueous intermediate to obtain a frozen solid; and
vii) lyophilize the frozen solid to obtain the encapsulated dimethyltryptamine.

Several pharmaceutical dosage forms can be prepared from the encapsulated microparticles or nanoparticles. Dosage forms can be prepared from the encapsulated microparticles or nanoparticles alone, or after mixing with other suitable excipients, fillers, etc. For example, the dosage forms may be prepared by suspending the microparticles or nanoparticles in a suitable liquid; compressing the microparticles or nanoparticles into a tablet; filling the microparticles or nanoparticles into a capsule to form a granulated capsules; or formulated into a soft or hard gel capsule. Optionally, tablets or capsules prepared in this way can be further coated with an additional polymer to further control release.

The invention is illustrated in more detail in the following two non-limiting examples.

### EXAMPLE 1

400 mg of Eudragit L-100 is dissolved in 20 mL of 95% ethanol, and 100 mg of DMT/5-MeO-DMT, or pharmaceutically acceptable salts or derivatives thereof, is added to the ethanolic solution while in agitation until complete dissolution is observed. A separate aqueous surfactant solution is prepared by dissolving 80 mg of Tween 80 in 80 mL of distilled water.

The alcoholic solution is loaded into a peristaltic pump and dripped into the aqueous solution, which is subjected to constant agitation by sonication or magnetic stirring. The resulting mixture is placed on a rotary evaporator until the ethanol is eliminated.

The resulting aqueous solution is frozen at -60°C and lyophilized.

### EXAMPLE 2

400 mg of Eudragit L-100 is dissolved in 20 mL of 95% ethanol, and 100 mg of DMT/5-MeO-DMT, or pharmaceutically acceptable salts or derivatives thereof, and 100 mg of curcumin are added to the ethanolic solution while in agitation until complete dissolution is observed. A separate aqueous solution of Tween 80 is prepared by dissolving 80 mg of Tween 80 in 80 mL of distilled water.

The alcoholic solution is loaded into a peristaltic pump and dripped into the aqueous solution, which is subjected to constant agitation by sonication or magnetic stirring. The resulting mixture is placed on a rotary evaporator until the ethanol is eliminated.

The resulting aqueous solution is frozen at -60°C and lyophilized.

### REFERENCES

1 Carbonaro T.M. and Gatch M.B., Brain Research Bulletin, 2016; 126:74-88: "Neuropharmacology of N, N-dimethyltryptamine".
2 Metzner R., New York: Thunder's Mouth Press, 1999: "Ayahuasca: hallucinogens, consciousness, and the spirit of nature".
3 McKenna D.J. et al., Journal of Ethnopharmacology, 1984; 10 (2):195-223: "Monoamine oxidase inhibitors in South American hallucinogenic plants: tryptamine and beta-carboline constituents of ayahuasca".
4 Strassman R.J., Journal of Psychoactive Drugs, 1991; 23:29-38: "Human hallucinogenic drug research in the United States: a present-day case history and review of the process".
5 Strassman R.J., The Journal of nervous and mental disease, 1995; 183:127-38: "Hallucinogenic drugs in psychiatric research and treatment. Perspectives and prospects".
6 Davis A.K. et al., The American Journal of Drug and Alcohol Abuse, 2019; 45:161-169: "5-Methoxy-N,N-Dimethyltryptamine (5-MeO-DMT) Used in a Naturalistic Group Setting Is Associated with Unintended Improvements in Depression and Anxiety".
7 Osório, F. de L. et al., Revista Brasileira de Psiquiatria, 2015, 37(1):13-20: "Antidepressant Effects of a Single Dose of Ayahuasca in Patients with Recurrent Depression: A Preliminary Report".
8 Dos Santos R.G. and Hallak, J.E.C., Neuroscience and biobehavioral reviews, 2020, 108:423-434: "Therapeutic use of serotoninergic hallucinogens: A review of the evidence and of the biological and psychological mechanisms".
9 Szara S., Experientia, 1956, 12:441-442: "Dimethyltryptamin: its metabolism in man; the relation to its psychotic effect to the serotonin metabolism".
10 Franzen F, and Gross H., Nature, 1965; 206:1052: "Tryptamine, N,N-dimethyltryptamine, N,N-dimethyl-5-hydroxytryptamine and 5-methoxytryptamine in human blood and urine".
11 Arturo A. et al., Journal of nuclear medicine, 2011; 52:970-977: "In Vivo Long-Term Kinetics of Radiolabeled N,N-Dimethyltryptamine and Tryptamine".
12 McKenna, D.J. et al., Neuropharmacology, 1990; 29:193-198: "Differential interactions of indolealkylamines with 5-hydroxytryptamine receptor subtypes".
13 Narasimhachari N. et al., Biological Psychiatry, 1971; 3:21-23: "N,N-dimethylated indoleamines in blood".
14 Oon M.C. et al., Psychopharmacology, 1977; 54:171-175: "Factors affecting the urinary excretion of endogenously formed dimethyltryptamine in normal human subj ects" .
15 Smythies J.R. et al, Biological Psychiatry, 1979; 14:549-556: "Identification of dimethyltryptamine and O-methylbufotenin in human cerebrospinal fluid by combined gas chromatography/mass spectrometry".
16 Sitaram B.R. et al., Analytical biochemistry, 1983; 128:11-20: "The ion-pair extraction, purification, and liquid chromatographic analysis of indolealkylamines in human urine".
17 Forsstrom T., Tuominen J. and Karkkainen J., Scandinavian journal of clinical and laboratory investigation, 2001; 61:547-556: "Determination of potentially hallucinogenic N-dimethylated indoleamines in human urine by HPLC/ESI-MS-MS".
18 Luethi, D. and Liechti, M.E., The international journal of neuropsychopharmacology, 2018; 21:926-931: "Monoamine transporter and receptor interaction profiles in vitro predict reported human doses of novel psychoactive stimulants and psychedelics".
19 Szabo, A. et al., PLoS One, 2014; 9:e106533: "Psychedelic N,N-dimethyltryptamine and 5-methoxy-N,N-dimethyltryptamine modulate innate and adaptive inflammatory responses through the sigma-1 receptor of human monocyte-derived dendritic cells".
20 Sitaram, B.R. et al.; Biochemical Pharmacology, 1987; 36:1509-1512: "In vivo metabolism of 5-methoxy-N, N-dimethyltryptamine and N,N-dimethyltryptamine in the rat".
21 Callaway, J.C., Journal of Psychoactive Drugs, 2005, 37(2):157-61, "Fast and Slow Metabolisers of Hoasca".
22 Bahman H. et al., Pharmaceutics, 2019, 11:129: "Challenges and Recent Progress in Oral Drug Delivery Systems for Biopharmaceuticals".
23 Mora-Huertasa, C.E. et al., International Journal of Pharmaceutics, 2010, 113-142: "Polymer-based nanocapsules for drug delivery".
24 Yoo et al., International Journal of Pharmaceutics, 2011, 403:262-267, "Eudragit nanoparticles for mucosal drug delivery".
25 Barzegar-Jalali, M., Powder Technology, 2012, 219:211-216: "Comparison of physicochemical characteristics and drug release of diclofenac sodium-eudragit® RS100 nanoparticles and solid dispersions".
26 Larit F. et al., Phytomedicine, 2018, 40:27-36: "Inhibition of human monoamine oxidase A and B by flavonoids isolated from two Algerian medicinal plants".
27 Khatri and Juvekar, Acid. Pharmacogn Mag. 2016, 12(2):S116-S120: "Kinetics of Inhibition of Monoamine Oxidase Using Curcumin and Ellagic".
28 Zhang Y, Li L, and Zhang, Basic Clin Pharmacol Toxicol., 2020, 227:243-253: "Curcumin in antidepressant treatments: An overview of potential mechanisms, pre-clinical/clinical trials and ongoing challenges".

## Claims

1. A composition comprising:
particles of:
i) N,N-dimethyltryptamine (DMT), 5-methoxy-N,N-dimethyltryptamine (5-MeO-DMT), or pharmaceutically acceptable salts or derivatives thereof; and
ii) a pH-sensitive polymer,
wherein the particles are nanoparticles or microparticles.

2. The composition of claim 1, wherein the composition comprises a therapeutically effective dose of the DMT or 5-MeO-DMT.

3. The composition of claim 1, wherein the composition further comprises an inhibitor of monoamine oxidase (MAOI).

4. The composition of claim 3, wherein the MAOI is selected from the group consisting of beta-carbolines, resveratrol, curcumin, flavonoids, quercetin, crysine, shawls, and pharmaceutically acceptable salts or derivatives thereof.

5. The composition of claim 1, wherein the weight ratio of i) to ii) is between 1:100 to 1:4.

6. The composition of claim 5, wherein the weight ratio of i) to ii) is between 1:10 to 1:4.

7. The composition of claim 1, wherein the pH-sensitive polymer is soluble at a pH of greater than 6.

8. The composition of claim 1, wherein the particles have a particle size distribution of between 70 nm to 200 µm.

9. The composition of claim 8, wherein the particles have a particle size distribution of between 70 nm and 300 nm.

10. The composition of claim 1, wherein the composition further comprises a surfactant.

11. The composition of claim 10, wherein the surfactant is Tween 80.

12. A method of treating a neurological and/or psychiatric disorder and/or inflammatory disorder in a patient in need thereof comprising administering said patient the composition of claim 1.

13. A method of preparing an encapsulated dimethyltryptamine, the method comprising:
i) dissolving a pH-sensitive polymer in one or more solvents to make a first solution;
ii) agitating said first solution while adding DMT, 5-MeO-DMT, or a pharmaceutically acceptable salt or derivative thereof to make a second solution;
iii) prepare a separate aqueous third solution, which can optionally include a dissolved surfactant;
iv) agitating the third solution while adding the second solution to the third solution to obtain a fourth solution;
v) evaporating the one or more solvents from the fourth solution to obtain an aqueous intermediate;
vi) freezing the aqueous intermediate at -60 °C to obtain a frozen solid; and
vii) lyophilizing the frozen solid to obtain the encapsulated dimethyltryptamine.

14. A pharmaceutical dosage form comprising the composition of claim 1.

15. The pharmaceutical dosage form of claim 14, wherein the pharmaceutical dosage from is selected from the group consisting of a suspension, a granulated capsule, a soft gel capsule, a hard gel capsule, and a tablet.
